Europäisches Patentamt

European Patent Office

Office européen des brevets

(19)

(11) **EP 0 818 950 B1**

(12) **EUROPÄISCHE PATENTSCHRIFT**

(45) Veröffentlichungstag und Bekanntmachung des Hinweises auf die Patenterteilung:
**30.01.2002 Patentblatt 2002/05**

(21) Anmeldenummer: **96909158.6**

(22) Anmeldetag: **01.04.1996**

(51) Int Cl.7: **A01N 37/16**

(86) Internationale Anmeldenummer:
**PCT/EP96/01424**

(87) Internationale Veröffentlichungsnummer:
**WO 96/31119 (10.10.1996 Gazette 1996/44)**

(54) **ORGANISCHE PEROXOVERBINDUNGEN ENTHALTENDES MITTEL ZUR INSTRUMENTENDESINFEKTION**

AGENTS FOR DISINFECTING INSTRUMENTS

PRODUITS POUR LA DESINFECTION D'INSTRUMENTS

(84) Benannte Vertragsstaaten:
**AT BE CH DK ES FI FR GB GR LI LU NL PT SE**

(30) Priorität: **04.04.1995 DE 19512588**

(43) Veröffentlichungstag der Anmeldung:
**21.01.1998 Patentblatt 1998/04**

(73) Patentinhaber: **Bode Chemie GmbH & Co.**
**D-22525 Hamburg (DE)**

(72) Erfinder:
• **WINTER-EXTRA, Susanne**
**D-22549 Hamburg (DE)**
• **ANKER, Willem**
**D-25474 Ellerbek (DE)**
• **LUX, Dorit**
**D-22393 Hamburg (DE)**
• **OSTERMEYER, Christiane**
**D-22559 Hamburg (DE)**
• **PAEDE, Johannes**
**D-21035 Hamburg (DE)**

(74) Vertreter: **Siewers, Gescha, Dr.**
**Harmsen & Utescher Rechts- und Patentanwälte**
**Alter Wall 55**
**20457 Hamburg (DE)**

(56) Entgegenhaltungen:
EP-A- 0 061 268          EP-A- 0 147 223
EP-A- 0 357 238          EP-A- 0 383 005
EP-A- 0 469 983          WO-A-94/15465
WO-A-95/32625           DE-A- 2 653 738
DE-A- 2 815 400          DE-A- 3 615 787
DE-B- 2 654 164          GB-A- 2 137 882
GB-A- 2 257 630          US-A- 5 010 109

• **S.S.BLOCK: "Disinfection, Sterilization and Preservation" 1991 , LEA & FEBIGER , PHILADELPHIA, US XP002012147 Kapitel 9: 'PEROXYGEN COMPOUNDS', Seite 167-181 siehe Seite 178, Spalte 1, Absatz 2 siehe Seite 179, Spalte 1, Absatz 4**
• **DATABASE WPI Section Ch, Week 9351 Derwent Publications Ltd., London, GB; Class D22, AN 93-408353 XP002021566 & JP 05 305 126 A (NIPPON OILS & FATS CO LTD) , 19.November 1993 & CHEMICAL ABSTRACTS, vol. 120, no. 12, 21.März 1994 Columbus, Ohio, US; abstract no. 144225,**
• **CHEMICAL ABSTRACTS, vol. 122, no. 11, 13.März 1995 Columbus, Ohio, US; abstract no. 125938, XP002021565 & JP 06 321 711 A (NIPPON OILS & FATS) 22.November 1994**

**Beschreibung**

Die Erfindung betrifft Mittel zur Instrumentendesinfektion.

Im Bereich der Desinfektion wird unterschieden zwischen Mitteln zur Hand- bzw. Hautdesinfektion, zur Flächendesinfektion und zur Instrumentendesinfektion, da an diese Mittel durchaus unterschiedliche Anforderungen gestellt werden. Zur Instrumentendesinfektion werden heute vorzugsweise Aldehyde eingesetzt, die aber verschiedene Nachteile aufweisen. Gemeinsam ist allen Aldehyden, daß sie hautreizend sind und da sich ein Kontakt zwischen Desinfektionsmittel bei der Instrumentendesinfektion und Haut häufig doch nicht vermeiden läßt, bei entsprechend disponierten Personen zu Hautschäden führen. Des weiteren wird allen Aldehyden mit relativ kurzer Alkylkette ein kanzerogenes Potential nachgesagt, so daß auch aus diesem Grunde ein Kontakt mit aldehydhaltigen Desinfektionsmitteln soweit wie möglich vermieden werden sollte, was sich aber häufig nicht durchführen läßt. Ein besonderer Nachteil der aldehydhaltigen Desinfektionsmittel liegt aber noch in der Geruchsbelästigung, die bei maschineller Behandlung von Instrumenten besonders intensiv auftritt und vom damit täglich in Berührung kommenden Personal in Kliniken und Praxen als besonders lästig empfunden wird.

Man hat daher bereits versucht, auf die Verwendung von Aldehyden zu verzichten und andere bekannte Desinfektionsmittel einzusetzen. Da Quats und Amine gerade bei maschineller Bearbeitung zu stark schäumen, ist die Möglichkeit, auf andere Desinfektionsmittel auszuweichen, stark eingeschränkt, da praktisch nur Guanidine, Phenole und chlorabspaltende Verbindungen in Frage kommen, wobei die letzteren Verbindungstypen selbst wieder neben anderen Nachteilen vor allem Geruchsbelästigungen bedingen.

- GB 2 257 630 beschreibt, dass zur Lagerung des Konzentrats gemäß dieser Erfindung das Konzentrat auch in einer wässrigen oder alkohol-wässrigen oder alkoholischen Lösung von niedermolekularen Alkoholen vorliegen kann (S. 7 Z. 35). Das erzeugt erhebliche Probleme, da diese Lösung relativ schnell verbraucht werden muss, weil sie instabil ist.

  Diese Entgegenhaltung zeigt daher die Aufgabe der vorliegenden Erfindung an, löst sie aber nicht.

- In der PCT/GB94/00010, um die Aufgabe der Erfindung zu lösen, wurde ein Verfahren vorgeschlagen, bei welchem zwei Komponente (die wässrige Lösung einer Persäure einerseits und die wässrige Lösung von Wasserstoffperoxid enthaltend Peroxistabilisatoren andererseits) vor dem Gebrauch gemischt werden. Damit wurde die Lösung der Aufgabe nicht nahegelegt.

- EP 0 147 223 beschreibt ein Desinfektionsmittel, enthaltend eine organische Peroxiverbindung, wobei das Desinfektionsmittelkonzentrat entweder in einer wässrigen Lösung vorliegt, und falls diese s Desinfektionsmittel als Schädlingsbekämpfungsmittel angewendet soll in einer Lösung mit einem organischem Lösungsmittel (S. 14, Beispiel 27) . Diese Lösung kann aber nicht mit Wasser gemischt oder in Wasser emulgiert werden. Eine Persäure ist hier nicht erwähnt. Der Fachmann erhält keinen Hinweis, ein Desinfektionsmittelkonzentrat in einer nichtwässrigen Lösung herzustellen, wenn er sie mit Wasser vermischen will, da die mit Wasser zu verdünnenden Konzentrate immer Wasser enthalten.

- PCT/US95/06070 beschreibt ein Desinfektionsmittel, das durch Mischen einer wässrigen Lösung von einem Ester und einer Lösung eines Oxidationsmittels unmittelbar vor dem Verbrauch hergestellt wird. Damit führt diese Entgegenhaltung von der erfindungsgemäß vorgeschlagenen Lösung sogar weg.

- US 5 010 109 beschreibt eine Zusammensetzung enthaltend ein Monoterpenhydroperoxid in einer alkoholischen (96% ethanolischen) Lösung, Beispiele 2-6, insbesondere Beispiel 2, in welchem über eine "stock solution" gesprochen wird, was einem Konzentrat (in der o.g. alkoholischen Lösung) gleichzustellen ist.

- EP 0 383 005 beschreibt ein Konzervierungsmittel für Systeme oder Produkte mit einer wässrigen Phase wie beispielsweise Farben, Lacke, Klebstoffe oder wässrige Emulsion wie Bohrölemulsion oder Kühlwasserkreisläufe. Daher bewegt sich diese Entgegenhaltung nicht auf dem Gebiet der Erfindung und ist für den Fachmann unbrauchbar.

Es besteht daher noch ein Bedarf nach einem Mittel für die Instrumentendesinfektion, das bei hervorragender Wirksamkeit gegen sämtliche Mikroorganismen und Sporen die geschilderten Nachteile, insbesondere bezüglich der Geruchsbelästigung, nicht aufweist.

Erfindungsgemäß werden Mittel zur Instrumentendesinfektion vorgeschlagen, die gekennzeichnet sind an einen Gehalt an organischen Peroxidbildnern. Organische Peroxidbildner sind an und für sich als Desinfektionsmittel bekannt. Ursprünglich wurden alle diese Verbindungen als Bleich- und Desinfektionsmittel in Waschmitteln eingesetzt, aber sie werden zunehmend auch im echten Desinfektionsbereich für die Flächendesinfektion benutzt. Peroxidbildner gelten aber als langsam wirkende Desinfektionsmittel, was bei der Flächendesinfektion, bei der die Lösungen Gelegenheit haben, stundenlang einzuwirken, keine Rolle spielt. Diese Eigenschaft von Peroxidbildnern sprach aber gegen die Anwendung als Mittel zur Instrumentendesinfektion, da hierbei höchstens von einer Einwirkungszeit von einer Stunde ausgegangen wird entsprechend den Richtlinien der deutschen Gesellschaft für Hygiene und Mikrobiologie.

Überraschenderweise hat sich jetzt herausgestellt, daß auch mit Peroxidbildnern eine Instrumentendesinfektion innerhalb der vorgegebenen Zeitspanne entsprechend den Richtlinien möglich ist und daß Desinfektionsmittel auf dieser Basis nicht nur bakterizid, sondern auch sporozid und viruzid wirksam sind. Ein besonderer Vorteil der erfindungsgemäßen Desinfektionsmittel liegt in der Tatsache, daß sie praktisch keinen Eigengeruch aufweisen, über eine außerwöhnlich breite Materialverträglichkeit verfügen und hervorragend biologisch abbaubar sind und damit im Gegensatz bspw. zu Phenolen fast keine Umweltbelastungen darstellen.

Als Peroxidbildner können organische Persäuren oder Alkyperoxide eingesetzt werden. Die organischen Persäuren können aliphatische oder aromatische Verbindungen sein und als solche oder in Form ihrer verträglichen Alkali-, Erdalkali- oder Ammoniumsalze vorliegen. Beispiele für aliphatische Persäuren, die einsetzbar sind, sind Perfettsäuren von Perpropionsäure bis zur Perlaurylsäure. Als aromatische Persäuren werden Perbenzolsäure, Perphthalsäure und andere Perbenzolsäuren eingesetzt wie bspw. p-Methoxyperbenzolsäure. Als besonders geeignet hat sich Magnesiummonoperphthalat erwiesen.

Als organische Peroxide können die Alkylperoxide mit gerader oder verzweigter Kette mit mittlerer Kettenlänge verwendet werden wie bspw. t-Butylperoxid oder ggf. auch hydroaromatische Verbindungen wie 1,1-Dioxy bis -Cyclohexanol.

Diese Verbindungen sind in der Regel in wasserfreiem Milieu bei Raumtemperatur stabil; so weist bspw. Magnesiummonoperphthalat nach 3-jähriger Lagerung noch über 90% Aktivität auf. Die Peroxide zerfallen aber zunehmend bei steigenden Temperaturen und spalten bei Temperaturen ab 50 bis 60 °C sehr schnell Sauerstoff ab. Für viele Anwendungszwecke ist es daher ausreichend, die organischen Perverbindungen als solche einzusetzen, da bei der Instrumentendesinfektion in vielen Fällen höhere Temperaturen als 50 bis 60°C zum Einsatz kommen. Falls aber bei der Kaltdesinfektion von bspw. Kunststoffmaterialien erwünscht oder erforderlich, kann der Zerfall der Perverbindungen durch Zusatz von Aktivatoren auch bei niedrigen Temperaturen gefördert werden. Geeignete Aktivatoren sind an und für sich bekannt; so können katalytisch wirkende Mengen an Eisen-, Kobalt- oder Mangansalzen eingesetzt werden oder *TAD oder *TAGU, die auch in der Waschmittelindustrie als Aktivatoren Verwendung finden.

Die erfindungsgemäßen Desinfektionsmittel können darüber hinaus übliche Zusatzstoffe enthalten wie bspw. Tenside wie Alkylbenzolsulfonate mit 9 bis 15 C-Atomen in gerader Kette, Alkylsulfonate mit 10 bis 24 C-Atomen in gerader Kette, Alkoholsulfate oder nichtionische Tenside wie Alkylphenol-Ethylenoxidaddukte oder Phosphatderivate des Ethylenoxids. Darüber hinaus können die Zubereitungen Dickungshilfsmitteln wie substituierte Zellulosen enthalten, die gleichzeitig als Dispergierhilfsmittel wirksam sind. Weitere Hilfsstoffe sind Korrosionsinhibitoren, Stellmittel und Mittel zur pH-Einstellung.

Der pH-Wert einer 1-%igen wässrigen Lösung sollte im Bereich zwischen pH 5 bis 9 und vorzugsweise um pH 7 liegen, da bei stärker saurem oder basischem pH das Material der Instrumente und Geräte auf Dauer geschädigt werden könnte.

Die erfindungsgemäßen Desinfektionsmittel können in Pulver- oder Granulatform vorliegen und bspw. in Dosierbeutel abgepackt werden, so daß dann die Gebrauchslösung frisch angesetzt werden kann. Flüssige Perverbindungen können in an sich aus der Waschmittelherstellung bekannter Weise zu Pulvern bzw, Granulaten durch Sprühtrocknung verarbeitet werden. Die Wirkstoffe können aber auch als Lösung oder Suspension in nicht wässrigen Lösungsmiteln verarbeitet werden und weisen dann ebenfalls eine hinreichende Lagerstabilität auf. Geeignete nicht wässrige, hautverträgliche Lösungsmittel sind bspw. Glyzerin oder Bernsteinsäure- oder Malonester, die beim Verdünnen hinreichend wasserlöslich sind bzw. durch den Tensidzusatz emulgierbar werden. Die festen oder flüssigen Zubereitungen können zwischen etwa 5 bis 95 Gewichtsprozent Wirkstoffe enthalten, wobei der Rest aus Hilfstoffen besteht.

Die Gebrauchskonzentration der wässrigen Lösung kann zwischen 0,025 bis 2,0 Gewichtsprozent betragen, je nach Keimart und Einwirkungsdauer.

Die Erfindung wird im folgenden anhand der Beispiele näher erläutert:

Beispiel 1

Herstellung von Granulaten

Magnesiummonoperphthalat im folgenden abgekürzt als MMPP bezeichnet, kann beispielsweise in Wirbel-

*TAD : Tetraacetylettylendiamin
*TAGU : Tetracetylglykoluril

schichttrockner unter Mitverwendung von Granulierhilfsmitteln granuliert werden. Vorzugsweise wird bei der Herstellung der Granulate ein Wirbelschichttrockner WSG200 der Firma Glatt eingesetzt. Dieses Gerät verfügt über die Möglichkeit einer Luftvortrocknung.

Als Granulierhilfsmittel können Verdickungsmittel wie z.B. Polyvinylpyrrolidon, abgekürzt PVP, und/oder Cellulosederivate wie Methylhydroxyethylcellulose, Propylcellulose oder ähnliche Typen eingesetzt werden. Es ist auch möglich, diese Verdickungsmittel zusammen mit Tensiden zu benutzen wie beispielsweise Cocamidpropylbetain, Alkylethercarbonsäuren, Sulfosuccinate und/oder Alkylpolyglykolether.

Die nachfolgend beschriebenen Mischungen wurden im Wirbelschichttrockner granuliert, wobei die Granuliertemperatur von etwa 30°C auf 50°C während des Vorganges anstieg; für eine MMPP-Menge von 150 kg ist eine Granulierzeit von ca. 2 Stunden notwendig.

Granulate wurden hergestellt aus folgenden Mischungen:

97,0 g MMPP + 3,0 g PVP

97,0 g MMPP + 1,0 g Xanthan

97,0 g MMPP + 1,0 g Xanthan + 2,0 g Ethercarbonsäure

90,0 g MMPP + 1,0 Methylhydroxylcellulose + 9,0 Cocamidpropylbetain

Beispiel 2

Herstellung von Tabletten

Die Tablettenherstellung aus MMPP erfolgt in an sich bekannter Weise durch Pressen von Granufaten oder Pulvern in geeigneten technischen Geräten. Als Tablettierhilfsmittel finden die üblichen Binde-, Spreng-, Gleit- und Füllmittel Verwendung. Besonders geeignet sind PVP, substituierte Cellulosen und ggf. Tenside wie beispielsweise Alkylpolyglycolether, Ethercarbonsäuren, Sulfosuccinate und/oder Betaine. Als Sprengmittel können auch Kombinationen aus Säure und Base wie beispielsweise Hydrogencarbonate und Säuren eingesetzt werden.

Die nachfolgenden Rezepturen wurden auf eine Fette EXI Tablettenmaschine mit einem Stempel 25 mm, facettiert gepreßt. Die Preßkraft sollte mindestens 22 KN betragen. Die eingesetzte Tablettiermaschine arbeitet mit einer Geschwindigkeit von über > 15 Hub/min.

Hergestellt wurden Tabletten aus folgenden Mischungen:

84,0 g MMPP + 8,0 g Polyethylenglykol + 7,0 g Alkylpolyglykolether + 1,0 g PVP

90,0 g MMPP + 1,0 g Methylhydroxyethylcellulose + 2,0 g PVP + 7,0 g Cocamidopropylbetain

25,0 g MMPP + 23,0 g Natriumhydrogencarbonat + 29,0 g Zitronensäure + 13,0 g Polyethylenglykol 400

Die angegebenen Substanzen wurden, wie in Beispiel 1 beschrieben, zu Granulaten verarbeitet und dann in an sich bekannter Weise tablettiert.

Beispiel 3

Herstellungen von Suspensionen oder Emulsionen

Zur Herstellung wasserfreier Suspensionen oder Emulsionen werden hautverträgliche Träger eingesetzt wie beispielsweise Paraffine, Silikonöle und/oder Isopropylpalmitat. Als Hilfsstoffe können PVP, substituierte Cellulosen, Siliziumdioxid in feinteiliger Form oder die oben bereits genannten Tenside eingesetzt werden. Die Herstellung der Mischungenerfolgt unter kräftigen Rühren.

Hergestellt wurden folgende Suspensionen:

5,0 g MMPP + 93,0 g Silikonöl + 2,0 g Natriumlaurylsulfat

5,0 g MMPP + 90,0 g Paraffin dünnflüssig + 5,0 g Cocamidopropylbetain

5,0 g MMPP + 7,5 g feinteiliges Siliziumdioxid (Aerosil ®) + 87,5 g Isopropylpalmitat

5,0 g MMPP + 7,5 g PVP + 87,5 g Isopropylmyristat

**Patentansprüche**

1. Aldehydfreies Desinfektionsmittelkonzentrat auf Basis einer organischen Persäure und/oder ihren physiologisch verträglichen Salzen in einem flüssigen wasserfreien Träger, **dadurch gekennzeichnet, daß** ein Träger aus der Gruppe der flüssigen Paraffine, Silikonöle, Ester aliphatischer Säuren oder mehrwertiger Alkohole vorliegt, der mit Wasser entweder mischbar ist oder eine Emulsion bildet.

2. Desinfektionsmittelkonzentrat nach Anspruch 1, **gekennzeichnet durch** einen Gehalt an Magnesiumperphthalat.

3. Desinfektionsmittelkonzentrat nach Anspruch 1 oder 2, **gekennzeichnet durch** einen Gehalt an 5 bis 95 Gew.-% Magnesiumperphthalat.

4. Desinfektionsmittelkonzentrat nach Anspruch 1 bis 3, **gekennzeichnet durch** einen Gehalt an 5 Gew.-% Magnesiumperphthalat.

5. Desinfektionsmittelkonzentrat nach Anspruch 1 bis 4, **gekennzeichnet durch** einen zusätzlichen Gehalt an Tensiden und/oder Emulgierhilfsmitteln.

6. Desinfektionsmittelkonzentrat nach Anspruch 1 bis 5, **gekennzeichnet durch** einen zusätzlichen Gehalt an Peroxidaktivatoren.

7. Verwendung des Desinfektionsmittelkonzentrates nach einem der Ansprüche 1 bis 6 zur Herstellung eines Desinfektionsmittels.

8. Verwendung des Desinfektionsmittels nach Anspruch 7 zur Desinfektion von Flächen und/oder Instrumenten.

**Claims**

1. An aldehyde-free disinfectant concentrate based on an organic peracid and/or its physiologically compatible salts in a liquid anhydrous medium, **characterised in that** the medium is from the group comprising liquid paraffins, silicone oils, esters of aliphatic acids or polyvalent alcohols and is either miscible with water or forms an emulsion.

2. A disinfectant concentrate according to claim 1, **characterised by** a content of magnesium perphthalate.

3. A disinfectant concentrate according to claim 1 or 2, **characterised by** a content of 5 wt.% to 95 wt.% of magnesium perphthalate.

4. A disinfectant concentrate according to claims 1 to 3, **characterised by** a content of 5 wt.% of magnesium perphthalate.

5. A disinfectant concentrate according to claims 1 to 4, **characterised by** an additional content of surfactants and/or emulsifying aids.

**6.** A disinfectant concentrate according to claims 1 to 5, **characterised by** an additional content of peroxide activators.

**7.** Use of the disinfectant concentrate according to any one of claims 1 to 6 for the preparation of a disinfectant.

**8.** Use of the disinfectant according to claim 7 for disinfecting surfaces and/or instruments.


**Revendications**

**1.** Désinfectant concentré se fondant sur un peracide organique et / ou ses sels physiologiquement tolérable sans des aldéhydes dans un substrat liquide anhydre, **caractérisé en ce qu'**un substrat provenant du groupe des paraffines liquides, huiles de silicone, esters des acides aliphatiques ou des alcools polyvalentes est présent étant miscible avec de l'eau ou formant une émulsion.

**2.** Désinfectant concentré selon la revendication 1, **caractérisé en ce qu'**il contient le perphthalate de magnésium.

**3.** Désinfectant concentré selon les revendications 1 ou 2, **caractérisé** en a qu'il contient 5 jusqu'au 95 pour cent en poids du perphthalate du magnésium.

**4.** Désinfectant concentré selon les revendications 1 à 3 **caractérisé en ce qu'**il contient 5 pour cent au poids du perphthalate du magnésium.

**5.** Désinfectant concentré selon les revendications 1 à 4 **caractérisé en ce qu'**il contient une teneur complémentaire en dérivés tensio-actifs et / ou agents auxiliaires pour émulsionnement.

**6.** Désinfectant concentré selon les revendications 1 à 5 **caractérisé en ce qu'**il contient une teneur complémentaire an activateurs de peroxide.

**7.** Utilisation du désinfectant concentré selon les revendications 1 à 6 pour la production d'un désinfectant.

**8.** Utilisation du désinfectant selon la revendication 7 pour la désinfection des surfaces ou des instruments.